# EUROPEAN PATENT SPECIFICATION

(11) **EP 2 926 787 B1**
(45) Date of publication and mention of the grant of the patent: **22.08.2018**
(21) Application number: 13859501.2
(22) Date of filing: 27.11.2013
(51) Int. Cl.: A61F 13/49, A61F 13/56

(54) **DISPOSABLE DIAPER**
WEGWERFWINDEL
COUCHE JETABLE

(30) Priority: 27.11.2012 JP 2012259164
(43) Date of publication of application: 07.10.2015
(73) Proprietor: Unicharm Corporation, Ehime 799-0111 (JP)
(72) Inventor: SAKAGUCHI, Satoru, Kanonji-shi Kagawa 769-1602 (JP); YAMANAKA, Yasuhiro, Kanonji-shi Kagawa 769-1602 (JP); SAWA, Kana, Kanonji-shi Kagawa 769-1602 (JP)
(74) Representative: Dolleymores
(86) International application number: PCT/JP2013/081859
(87) International publication number: WO 2014/084233

(56) References cited:
- EP-A1- 2 810 631
- WO-A1-2013/031157
- JP-A- H09 253 130
- JP-A- 2002 000 648
- JP-A- 2002 165 831
- JP-A- 2004 215 694
- JP-A- 2004 215 694
- JP-A- 2006 122 453
- JP-A- 2010 029 532
- JP-A- 2010 507 417
- JP-A- 2012 192 244

## Description

### [Technical Field]

The present invention relates to a disposable diaper.

### [Background Art]

Conventionally, there is known a disposable diaper having: a front waistline region; a back waistline region; and a crotch region which is positioned between the front waistline region and the back waistline region, a pair of leg-hole opening units being formed therein, the disposable diaper being provided with an absorber running across the crotch region and extending to the front waistline region and the back waistline region.

Here, in Patent Literature 1, there is described the disposable diaper as mentioned above, in which a fastening tape is composed of a transparent film, and when the disposable diaper as mentioned above is worn by a wearer, the disposable diaper is structured so that a wearing assistant can visually recognize a target tape (refer to Patent Literature 1).

### [Citation List]

### [Patent Literature]

EP2810631 A1 and WO 2013/031157 A1 are citations under A54(3) EPC. They disclose a disposable diaper with a fastening tape which includes a base sheet made of nonwoven fabric and a hook sheet provided with a plurality of engagement hooks. An outer surface of the base sheet, which is opposite to a surface on which the hook sheet is provided, and an outer surface of the absorbent main body may be made of a nonwoven fabric having the same embossing pattern and fiber type. The base sheet may be configured by one layer of nonwoven fabric or a plurality of layers of nonwoven fabric layered on each other.

JP 2004 215694 A provides a disposable diaper in which a top sheet, a back sheet and a fastening tape substrate respectively have embossed surfaces, together with horizontal flat surfaces on the opposite sides of the embossed surfaces.

[PTL 1]Japanese Unexamined Patent Application Publication No. H05-507753

### [Summary of Invention]

Here, a structure of the fastening tape in the disposable diaper as mentioned above (a structure which is capable of visually recognizing a target tape) is formed with the emphasis on usability.

On the other hand, in recent years, there has been a growing demand from users (purchasers) for disposable diapers, and in particular, there has been a growing desire from the users to reduce a burden on a skin of an infant as much as possible.

However, in the disposable diaper as mentioned above, the fastening tape imparts a large sense of discomfort in texture or appearance in comparison with another portion of the disposable diaper that is formed of a nonwoven cloth, and for users who have a growing demand or desire for disposable diapers, there has been a problem that it is mistakenly recognized that the fastening tape is a foreign matter.

Accordingly, the present invention has been made in view of such a circumstance, and it is an object of the present invention to provide a disposable diaper which is capable of imparting an impression that a visual sense of integrity is felt between the fastening tape and another portion which is formed of a nonwoven cloth.

The present invention provides the disposable diaper of independent claim 1. The dependent claims specify preferred but optional features.

### [Brief Description of Drawings]

[Fig. 1] Fig. 1 is an exploded plan view (a skin contact surface side) of a disposable diaper according to an embodiment of the present invention.
[Fig. 2] Fig. 2 is an exploded plan view (a non-skin contact surface side) of the disposable diaper according to the embodiment of the present invention.
[Fig. 3] Fig. 3 is an enlarged view of a vicinity of a fastening tape in the disposable diaper according to the embodiment of the present invention shown in Fig. 2.
[Fig. 4] Fig. 4 is a sectional view of the disposable diaper, taken along the line F-F shown in Fig. 3.

### [Description of Embodiments]

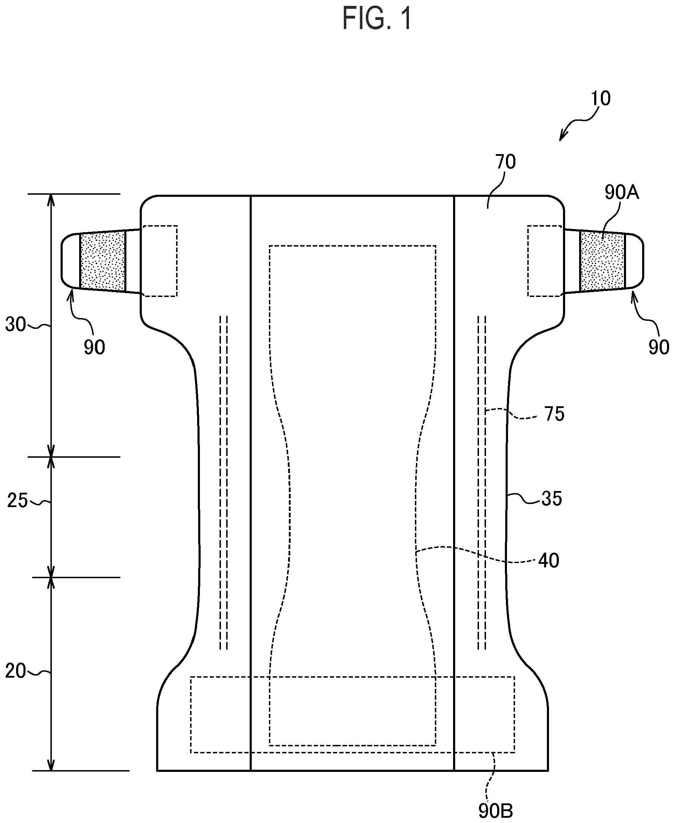

With reference to Fig. 1 to Fig. 4, a disposable diaper 10 according to a first embodiment of the present invention will be described.

It is noted that, in the description of the drawings below, identical or similar symbols are assigned to identical or similar portions. However, it should be noted that the drawings are schematic and ratios of respective dimensions and the like are different from actual ones.

Therefore, a specific dimension and the like should be determined in view of the following description. Moreover, among the drawings, the respective dimensional relations or ratios may differ.

It is to be noted that although explanation is made citing a tape-type disposable diaper 10 as an example in the present embodiment, the present invention is also applicable to a pant-type disposable diaper 10.

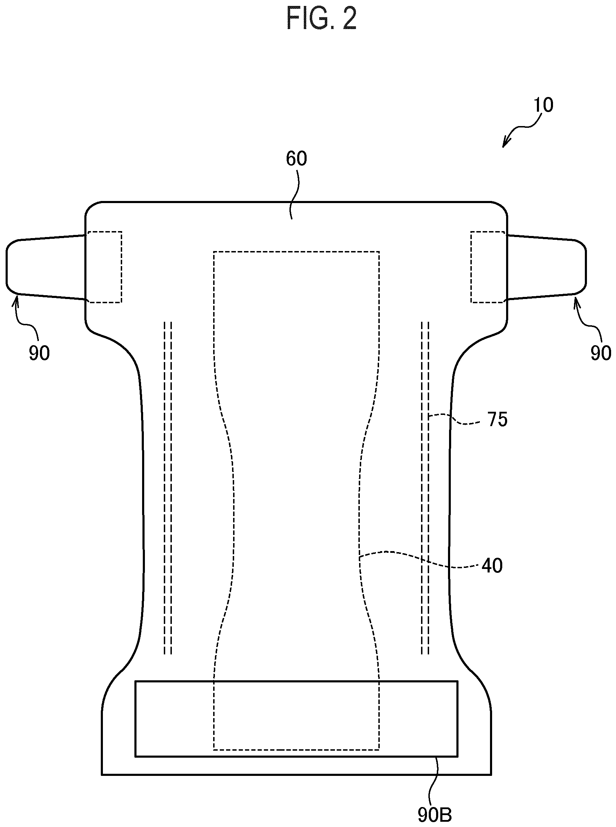

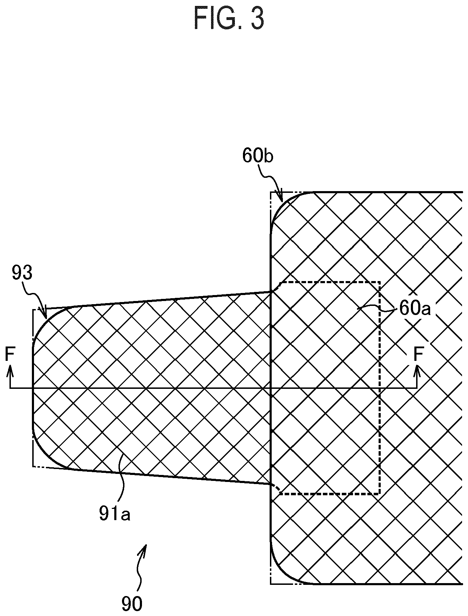

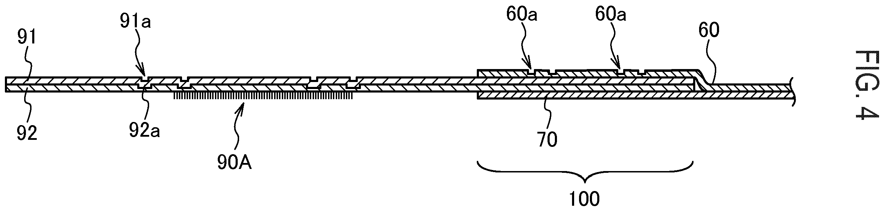

Fig. 1 is an exploded plan view (a skin contact surface side) of a disposable diaper according to an embodiment of the present invention. Fig. 2 is an exploded plan view (a non-skin contact surface side) of the disposable diaper according to the embodiment of the present invention. Fig. 3 is an enlarged view of a vicinity of a fastening tape in the disposable diaper according to the embodiment of the present invention shown in Fig. 2.. Fig. 4 is a sectional view of the disposable diaper, taken along the line F-F shown in Fig. 3..

As illustrated in Fig. 1 and Fig. 2, the disposable diaper 10 according to the present embodiment includes a front waistline region 20, a crotch region 25, and a back waistline region 30. Further, a pair of leg hole opening units 35 is formed in the disposable diaper 10.

Here, the front waistline region 20 is the portion that is in contact with the front waistline portion of the wearer, the back waistline region 30 is the portion that is in contact with a back waistline portion of the wearer, and the crotch region 25 is the portion that positioned between the front waistline region 20 and the back waistline region 30.

In the present embodiment, the direction from the front waistline region 20 towards the back waistline region 30 is called the product longitudinal direction L, and the direction perpendicular to the product longitudinal direction L is called the product widthwise direction W.

The disposable diaper 10 according to present embodiment includes an absorber 40 spanning the crotch region 25 and extending toward the front waistline region 20 and the back waistline region 30. The absorber 40 is configured by an absorbent core and a core wrap.

The absorber core is same as in the conventional disposable diaper, and can be configured appropriately by using popular components and materials, such as ground pulp and high absorbent polymer. The absorber core is wrapped by the sheet-like core wrap.

The core wrap is a sheet for wrapping the absorber core. A part of at least the skin surface contact side of the core wrap is configured by various fibrous nonwoven fabrics or a tissue sheet having liquid-permeability.

For example, an air-through fibrous nonwoven cloth, a spunbond nonwoven cloth, or an SMS (spunbond - meltblown - spunbond) nonwoven cloth having a mass of approximately 10 to 30 g/m², or a tissue sheet having a mass of approximately 10 to 30 g/m² can be used as the component configuring the core wrap.

Further, as shown in Fig. 1, a pair of leg stretching units (leg gathers) 75 are formed along the leg hole opening units 35 and are stretchable in at least the product longitudinal direction L.

For example, the leg stretching units 75 each may be composed of a predetermined number of elastic members (two elastic members in the example of Fig. 1). Also, the leg stretching units 75 each may be formed of a stretchable sheet member.

Also, a topsheet (not shown) which is a transparent nonwoven cloth sheet is provided at a surface side (a skin contact surface side) of an absorber 40, and a backsheet 60 which is composed of a nonwoven cloth sheet is provided at a back face side (a non-skin contact surface side) of the absorber 40.

Furthermore, a liquid-impermeable leakage preventing sheet (not shown) is provided between the topsheet 50 and the backsheet 60.

A side flap 70 is provided at both side edges in the product widthwise direction W of the absorber 40. The side flaps 70 are made of one or two or more pieces of nonwoven fabrics overlapping one another.

Also, as shown in Fig. 1 and Fig. 2, the disposable diaper 10 according to the embodiment is provided with a pair of fastening tapes 90 configured to extend to the outside of the product widthwise direction W from both side edges of the disposable diaper 10 in the front waistline region 20 (or the back waistline region 30).

In addition, the disposable diaper 10 according to the embodiment is provided with a target tape 90B in a predetermined region of the non-skin contact surface side of the back waistline region 30 (or the front waistline region 20).

Here, as shown in Fig. 1 and Fig. 4, at the fastening tape 90, a hook unit 90A is provided, and the hook unit 90A of the fastening tape 90 is mounted to the target tape 90B, whereby the disposable diaper 10 can be retained on the wearer's body.

Here, the fastening tape 90 is composed of a plurality of nonwoven cloth sheets. As shown in Fig. 4, in the disposable diaper 10 according to the embodiment, the fastening tapes 90 are composed of two nonwoven cloth sheets 91 and 92.

In addition, as shown in Fig. 3 and Fig. 4, at both of the side edges as mentioned above, embossment 60a is provided at the backsheet 60 (a first nonwoven cloth sheet) which is provided at the most non-skin contact surface side of a respective one of the nonwoven cloth sheets (the backsheet 60 and the side flaps 70) that correspond to a joint region 100 in which the fastening tapes 90 is joined.

On the other hand, as shown in Fig. 3 and Fig. 4, embossment 91a having a same shape as or a similar shape to that of the embossment 60a that is provided at the backsheet 60 (the first nonwoven cloth sheet) in a nonwoven cloth sheet 91 which is provided at the most non-skin contact surface side of the respective one of the nonwoven cloth sheets 91 and 92 that constitute the fastening tapes 90.

It is to be noted that the same shape of embossment denotes that patterns which are formed of embossments 60a and 91a and/or dimensions which constitute the patterns (such as areas of the respective lattices) are the same as each other.

It is also to be noted that the similar shape of embossment denotes that, although the patterns formed of embossment 60a and embossment 91a are the same as each other, dimensions of shapes which constitute the patterns are different from each other and/or either width or height is stretched or shrunk while the shapes (for example, a triangle or a square) are maintained.

Alternatively, the backsheet (the first nonwoven cloth sheet) and the nonwoven cloth sheet 91 may be composed of a nonwoven cloth which is generated by a same kind of manufacturing method.

In addition, as shown in Fig. 4, embossment 92a which is different from the embossment 91a that is provided with respect to the nonwoven cloth sheet 91 is provided at the nonwoven cloth sheet 92 other than the nonwoven cloth sheet 91 that constitutes the fastening tapes 90.

Further, as shown in Fig. 3, it may be that corners 93 in the fastening tapes 90 that extend from both side edges of the disposable diaper each are cut in an arc shape, and that corners 60b at both side edges of the disposable diaper each are cut in an arc shape.

Furthermore, it is preferable that a value of KES bending rigidity per unit length in the product longitudinal direction L all over the fastening tape 90 and a value of KES bending rigidity per unit length in the product widthwise direction W all over the fastening tape 90 each are adapted to be smaller than 1.45 gf • cm²/cm.

The flexural properties were measured by using KES-FB2 manufactured by KATO TECH CO., LTD., by fixing each sample (such each sample obtained by cutting out the fastening tape 90 together with the nonwoven cloth sheets 91 and 92) between the chucks (while facing down the skin contact surface side (the inside) of the fastening tape 90 in the disposable diaper 10), bending to the front up to maximum curvature +2.5 cm⁻¹, and then bending to the back up to maximum curvature -2.5 cm⁻¹, and then returning to the origin.

The value of KES bending rigidity can be set to be smaller than 1.45 gf • cm²/cm by employing a plurality of nonwoven cloth sheets which constitute the fastening tapes 90 or by controlling types of the respective nonwoven cloth sheets or embossment rate forming a pattern or the like.

Further, at the target tape 90B, there may be provided embossment having a similar shape to that of the embossment 91a that is provided at a respective one of the backsheet (the first nonwoven cloth sheet) and the nonwoven cloth sheet 91.

Here, each nonwoven cloth sheet is formed by laminating single-layered fibers, more preferably by laminating multi-layered fibers.

The target tape 90B is composed of a plurality of nonwoven cloth sheets like the fastening tapes 90 or the backsheet 60.

In this manner, at the time of wearing the disposable diaper 10, when the fastening tape 90 is mounted to the target tape 90B, a visually integrated pattern is formed all over the front waistline region 20 and the back waistline region 30, making it possible to impart a good impression that any element as a foreign matter is not present.

Namely, nonwoven cloth sheets which constitute the target tapes 90B are formed by laminating multi-layered fibers, whereby the durability and engagement property that are required for the target tape 90B are easily achieved while embossment of a same or similar shape are provided.

It is to be noted that, in a case where the exterior sheet is provided at the non-skin contact surface side of the backsheet 60, the first nonwoven cloth sheet that is provided at the most non-skin contact surface side of a respective one of the nonwoven cloth sheets that correspond to the joint region 100 is obtained as the exterior sheet in place of the backsheet 60.

In so far as the disposable diaper 10 according to the embodiment is concerned, the embossment 91a having a same as or a similar shape to that of the embossment 60a that is provided at the backsheet 60 (the first nonwoven cloth sheet) is provided at the nonwoven cloth sheet 91 that is provided at a respective one of the most non-skin contact surface side of the respective one of the nonwoven cloth sheets 91 and 92 that constitute the fastening tapes 90, thus making it possible to impart an impression that a visual sense of integrity is felt between a respective one of the fastening tapes 90 and the backsheet 60 (or the exterior sheet).

Also, the fastening tapes 90 each, as shown in Fig. 4, may be a structure which is sandwiched between the backsheet 60 and a respective one of the side flaps 70 or may be structured so as to be joined with an interior face of the respective one of the side flaps 70.

According to the structure as mentioned above, the fastening tapes 90 are not released out to the non-skin contact surface side of the backsheet 60; and therefore, an impression of a visual sense of integrity all over the front waistline region 20 and the back waistline region 30 is improved more remarkably.

In addition, the nonwoven parts of the non-skin contact surface sides of the backsheets 60 and the fastening tapes 90 are composed of a crimped texture.

This is constituted by employing a crimped fiber. As the crimped fiber, for example, there can be employed a fiber obtained by employing a heat shrink crimping approach. In a case where the heat shrink crimping approach is employed, a composite fiber according to a side-by-side type or a core-clad type made of polypropylene and polyethylene, for example, is spun, and then crimping can be developed by a heat shrink difference due to a difference in the melting point.

In particular, it is preferable to employ a composite fiber which is obtained by a heat shrink crimping approach according to an eccentric core-clad type while polypropylene is employed as a core and copolymer of ethylene/propylene is employed as a clad, since a desired crimping count, a crimping rate, a crimping and stretching rate, a crimping and restoring rate or the like can be adjusted.

By employing such a crimped fiber, the bulkiness of the fiber is easily developed, and an image which is closer to a cloth (in particular, a kilting-like cloth in the embodiment) can be exhibited. In addition, together with embossment, an image which is further closer to a cloth (a kilting-like cloth) can be exhibited.

As described above, the present invention has been described in detail by using the above-described embodiment. However, it is apparent to those skilled in the art that the present invention is not limited to the embodiment described in this description. The present invention can be modified and changed without departing from the gist and the scope of the present invention defined by the appended claims. Therefore, the description is aimed at illustration and explanation of the present invention, and does not have any meaning which limits the present invention.

### [Industrial Applicability]

It is possible to provide a disposable diaper which is capable of imparting an impression that a visual sense of integrity is felt between a respective one of fastening tapes and another portion which is formed of a nonwoven cloth.

### [Reference Signs List]

10... Disposable diaper
20... Front waistline region
25... Crotch region
30... Back waistline region
35... Leg hole opening unit
40... Absorber
60... Backsheet
70... Side flap
75... Leg stretching unit
90... Fastening tape
90B... Target tape

## Claims

1. A disposable diaper (10) having: a front waistline region (20); a back waistline region (30); and a crotch region (25) which is positioned between the front waistline region and the back waistline region,
the disposable diaper comprising an absorber (40) running across the crotch region and extending to the front waistline region and the back waistline region, and the disposable diaper having: a product longitudinal direction (L) which is oriented from the front waistline region to the back waistline region and a product widthwise direction (W) which is orthogonal to the product longitudinal direction,
wherein a pair of fastening tapes (90) are provided so as to extend from both side edges of the disposable diaper in either the front waistline region or the back waistline region to an outside in the product widthwise direction,
wherein the fastening tapes each are composed of a plurality of nonwoven cloth sheets (91, 92),
wherein embossment (60a) is provided at a first nonwoven cloth sheet (60) which is provided at a most non-skin contact surface side of a respective one of nonwoven cloth sheets which correspond to a joint region (100) in which the fastening tapes are joined in both of the side edges,
wherein embossment (91a) having a same as or a similar shape to a shape of the embossment (60a) that is provided at the first nonwoven cloth sheet (60) is provided at a second nonwoven cloth sheet (91) which is provided at the most non-skin contact surface side of the respective one of the nonwoven cloth sheets (91, 92) that constitute the fastening tapes (90),
wherein a similar shape of embossment denotes that patterns formed of embossment (60a), which is provided at the first nowoven cloth sheet (60), and embossment (91a), which is provided at the second nowoven cloth sheet (91), are the same as each other, while the dimensions of the shapes which constitute the patterns are different from each other and/or either width or height is stretched or shrunk while the shapes are maintained,
wherein embossment (92a) different from the embossment (91a) that is provided with respect to the second nonwoven cloth sheet (91) is provided at the nonwoven cloth sheet (92) other than the second nonwoven cloth sheet that constitute the fastening tapes,
wherein a nonwoven cloth at a non-skin contact surface side of a respective one of the fastening tape (90) is composed of a crimped fiber, and
wherein a backsheet (60) is provided at a non-skin contact surface side of the absorber (40), and wherein a nonwoven cloth at a non-skin contact surface side of the backsheet is composed of a crimped fiber.

2. The disposable diaper according to claim 1, wherein corners (93) at the fastening tapes (90) extending from both of the side edges each are cut in an arc shape, and wherein the corners (60b) at both of the side edges of the disposable diaper each are cut in an arc shape.

3. The disposable diaper according to claim 1 or claim 2, wherein a value of KES bending rigidity per unit length in the product longitudinal direction L all over the fastening tape and a value of KES bending rigidity per unit length in the product widthwise direction W all over the fastening tape each are adapted to be smaller than 1.45 gf • cm²/cm and are measured as described in the description.

4. The disposable diaper according to any one of claims 1 to 3, wherein a target tape (90B) is provided in a region in which the fastening tapes are not provided, of the front waistline region and the back waistline region, and wherein at the target tape, there is provided embossment having a similar shape to a shape of the embossment that is provided at the first nonwoven cloth sheet and the second nonwoven cloth sheet.

5. The disposable diaper according to claim 4, wherein the target tape (90B) is composed of a nonwoven cloth sheet which is formed by laminating multi-layered fibers.

## Patentansprüche

1. Einwegwindel (10), die Folgendes aufweist: einen vorderen Taillenbereich (20), einen hinteren Taillenbereich (30) und einen Schrittbereich (25), der zwischen dem vorderen Taillenbereich und dem hinteren Taillenbereich positioniert ist,
wobei die Einwegwindel einen Absorber (40) umfasst, der über den Schrittbereich verläuft und sich bis zu dem vorderen Taillenbereich und dem hinteren Taillenbereich erstreckt, und wobei die Einwegwindel Folgendes aufweist: eine Produktlängsrichtung (L), die von dem vorderen Taillenbereich zu dem hinteren Taillenbereich ausgerichtet ist, und eine Produktbreitenrichtung (W), die senkrecht zu der Produktlängsrichtung vorliegt,
wobei ein Paar an Verschlussbändern (90) derart bereitgestellt ist, dass sie sich von beiden Seitenrändern der Einwegwindel in entweder dem vorderen Taillenbereich oder dem hinteren Taillenbereich zu einer Außenseite in der Produktbreitenrichtung erstrecken,
wobei die Verschlussbänder jeweils aus einer Vielzahl von Vliesstofflagen (91, 92) bestehen,
wobei eine Prägung (60a) auf der ersten Vliesstofflage (60) bereitgestellt ist, die auf einer möglichsten Nicht-Hautkontaktoberflächenseite einer jeweiligen von den Vliesstofflagen bereitgestellt ist, die einem Verbindungsbereich (100) entsprechen, in dem die Verschlussbänder an beiden Seitenrändern verbunden sind,
wobei eine Prägung (91a), die eine gleiche oder eine ähnliche Form wie eine Form der Prägung (60a) aufweist, die auf der ersten Vliesstofflage (60) bereitgestellt ist, auf einer zweiten Vliesstofflage (91) bereitgestellt ist, die an der möglichsten Nicht-Hautkontaktoberflächenseite der jeweiligen von den Vliesstofflagen (91, 92) bereitgestellt ist, die die Verschlussbänder (90) darstellen,
wobei eine ähnliche Form der Prägung anzeigt, dass Muster, die durch Prägung (60a), die auf der ersten Vliesstofflage (60) bereitgestellt ist, und Prägung (91a), die auf der zweiten Vliesstofflage (91) bereitgestellt ist, gebildet sind, einander gleich sind, während sich die Abmessungen der Formen, die die Muster darstellen, voneinander unterscheiden und/oder Breite oder Höhe gestreckt oder geschrumpft ist, während die Formen erhalten bleiben,
wobei eine Prägung (92a), die sich von der Prägung (91a) unterscheidet, die in Bezug auf die zweite Vliesstofflage (91) bereitgestellt ist, auf der Vliesstofflage (92) bereitgestellt ist, die nicht die zweite Vliesstofflage ist, die die Verschlussbänder darstellen,
wobei ein Vliesstoff auf der Nicht-Hautkontaktoberflächenseite eines jeweiligen des Verschlussbands (90) aus einer gekräuselten Faser besteht und
wobei eine hintere Lage (60) auf einer Nicht-Hautkontaktoberflächenseite des Absorbers (40) bereitgestellt ist und wobei ein Vliesstoff auf einer Nicht-Hautkontaktoberflächenseite der hinteren Lage aus einer gekräuselten Faser besteht.

2. Einwegwindel nach Anspruch 1, wobei Ecken (93) an den Verschlussbändern (90), die sich von beiden der Seitenränder erstrecken, jeweils in einer Bogenform geschnitten sind und wobei die Ecken (60b) an beiden der Seitenränder der Einwegwindel jeweils in einer Bogenform geschnitten sind.

3. Einwegwindel nach Anspruch 1 oder Anspruch 2, wobei ein KES-Wert der Biegesteifigkeit pro Einheitslänge in der Produktlängsrichtung L in dem gesamten Verschlussband und ein KES-Wert der Biegesteifigkeit pro Einheitslänge in der Produktbreitenrichtung W in dem gesamten Verschlussband jeweils angepasst sind, damit sie kleiner als 1,45 gf·cm²/cm sind, und wie in der Beschreibung beschrieben gemessen werden.

4. Einwegwindel nach einem der Ansprüche 1 bis 3, wobei ein Zielband (90B) in einem Bereich, in dem die Verschlussbänder nicht bereitgestellt sind, des vorderen Taillenbereichs und des hinteren Taillenbereichs bereitgestellt ist und wobei auf dem Zielband eine Prägung bereitgestellt ist, die eine ähnliche Form wie eine Form der Prägung aufweist, die auf der ersten Vliesstofflage und der zweiten Vliesstofflage bereitgestellt ist.

5. Einwegwindel nach Anspruch 4, wobei das Zielband (90B) aus einer Vliesstofflage besteht, die durch Laminieren von mehrschichtigen Fasern ausgebildet ist.

## Revendications

1. Couche jetable (10) ayant : une région avant au niveau de la taille (20) ; une région arrière au niveau de la taille (30) ; et une région au niveau de l'entrejambe (25) qui est positionnée entre la région avant au niveau de la taille et la région arrière au niveau de la taille,
la couche jetable comportant un élément absorbant (40) allant en travers de la région au niveau de l'entrejambe et s'étendant jusqu'à la région avant au niveau de la taille et la région arrière au niveau de la taille, et la couche jetable ayant : une direction allant dans le sens longitudinal du produit (L) qui est orientée pour aller de la région avant au niveau de la taille à la région arrière au niveau de la taille et une direction allant dans le sens de la largeur du produit (W) qui est orthogonale par rapport à la direction allant dans le sens longitudinal du produit,
dans laquelle une paire de rubans d'attache (90) sont mis en oeuvre de manière à s'étendre depuis les deux bords latéraux de la couche jetable dans soit la région avant au niveau de la taille soit la région arrière au niveau de la taille vers une partie extérieure dans la direction allant dans le sens de la largeur du produit,
dans laquelle les rubans d'attache sont chacun composés à partir d'une pluralité de feuilles en tissu non tissé (91, 92),
dans laquelle un gaufrage (60a) est mis en oeuvre au niveau d'une première feuille de tissu non tissé (60) qui est mise en oeuvre au niveau d'un côté formant surface le plus non en contact avec la peau d'une feuille respective parmi les feuilles de tissu non tissé qui correspondent à une région d'assemblage (100) dans laquelle les rubans d'attache sont reliés dans les deux bords latéraux,
dans laquelle un gaufrage (91a) ayant une forme identique ou une forme similaire à une forme du gaufrage (60a) qui est mis en oeuvre au niveau de la première feuille de tissu non tissé (60) est mis en oeuvre au niveau d'une deuxième feuille de tissu non tissé (91) qui est mise en oeuvre au niveau d'un côté formant surface le plus non en contact avec la peau d'une feuille respective parmi les feuilles de tissu non tissé (91, 92) qui constituent les rubans d'attache (90),
dans laquelle une forme similaire du gaufrage indique que les motifs formés au moyen du gaufrage (60a), qui est mis en oeuvre au niveau de la première feuille de tissu non tissé (60), et au moyen du gaufrage (91a), qui est mis en oeuvre au niveau de la deuxième feuille de tissu non tissé (91), sont identiques les uns par rapport aux autres, alors que les dimensions des formes qui constituent les motifs sont différentes les unes des autres et/ou soit la largeur soit la hauteur est étirée ou rétrécie alors que les formes sont maintenues,
dans laquelle le gaufrage (92a) différent du gaufrage (91a) qui est mis en oeuvre par rapport à la deuxième feuille de tissu non tissé (91) est mis en oeuvre au niveau de la feuille de tissu non tissé (92) autre que la deuxième feuille de tissu non tissé qui constituent les rubans d'attache,
dans laquelle un tissu non tissé au niveau d'un côté formant surface non en contact avec la peau d'un ruban respectif du ruban d'attache (90) est composé à partir d'une fibre frisée, et
dans laquelle une feuille de support (60) est mise en oeuvre au niveau d'un côté formant surface non en contact avec la peau de l'élément absorbant (40), et dans laquelle un tissu non tissé au niveau d'un côté formant surface non en contact avec la peau de la feuille de support est composé à partir d'une fibre frisée.

2. Couche jetable selon la revendication 1, dans laquelle des coins (93) des rubans d'attache (90) s'étendant depuis les deux bords latéraux sont chacun découpés en forme d'arc, et dans laquelle les coins (60b) au niveau des deux bords latéraux de la couche jetable sont chacun découpés en forme d'arc.

3. Couche jetable selon la revendication 1 ou la revendication 2, dans laquelle une valeur de rigidité de courbure KES par unité de longueur dans la direction allant dans le sens longitudinal du produit L sur la totalité du ruban d'attache et une valeur de rigidité de courbure KES par unité de longueur dans la direction allant dans le sens de la largeur du produit W sur la totalité du ruban d'attache sont chacune adaptées pour être inférieures à 1,45 gf · cm²/cm et sont mesurées tel qu'il est décrit dans la description.

4. Couche jetable selon l'une quelconque des revendications 1 à 3, dans laquelle un ruban cible (90B) est mis en oeuvre dans une région dans laquelle les rubans d'attache ne sont pas mis en oeuvre, parmi la région avant au niveau de la taille et la région arrière au niveau de la taille, et dans laquelle est mis en oeuvre, au niveau du ruban cible, un gaufrage ayant une forme similaire à une forme du gaufrage qui est mis en oeuvre au niveau de la première feuille de tissu non tissé et de la deuxième feuille de tissu non tissé.

5. Couche jetable selon la revendication 4, dans laquelle le ruban cible (90B) est composé à partir d'une feuille de tissu non tissé qui est formée par le contrecollage de fibres à couches multiples.
